(19) Europäisches Patentamt
European Patent Office
Office européen des brevets

(11) **EP 1 867 990 A1**

(12) **EUROPEAN PATENT APPLICATION**

(43) Date of publication:
**19.12.2007 Bulletin 2007/51**

(51) Int Cl.:
*G01N 33/543* (2006.01)  *G01N 33/58* (2006.01)

(21) Application number: **06115479.5**

(22) Date of filing: **14.06.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Koninklijke Philips Electronics N.V.
5621 BA  Eindhoven (NL)**

(72) Inventor: **The designation of the inventor has not yet been filed**

(74) Representative: **Van Velzen, Maaike Mathilde
Philips
Intellectual Property & Standards
P.O. Box 220
5600 AE Eindhoven (NL)**

(54) **Sensitive assay through amplification of a label signal**

(57)     This invention relates to a device and a method for amplifying a signal generated from primary nanoparticle labels in an assay by using secondary nanoparticle labels, typically magnetic labels, wherein by binding the secondary labels to the primary labels the results in that the signal produced from the labels will be amplified.

Fig. 1

EP 1 867 990 A1

**Description**

FIELD OF THE INVENTION

The present invention relates to a device and a method for amplifying a signal generated from primary nanoparticle labels in an assay. The present invention further relates to relates to a use of a combination of a primary and secondary nanoparticle labels for amplifying a signal generated from the primary nanoparticle label in an assay.

BACKGROUND OF THE INVENTION

A part of health care research involves developing easy to use molecular diagnostics, for molecules such as DNA, RNA, proteins, peptides, hormones, metabolites, drugs etc as well as to determine the activity and function of active and catalytic biomolecules such as, prions, enzymes, aptamers, ribozymes, and deoxyribozymes and to identify cells including human tissue, human cells, bacteria and viruses.

Magnetic biosensors are used for detecting biological targets labeled with superparamagnetic particles. Several label formats can be implemented for the detection of biological analyte. These include the direct assay, in which a labeled analyte binds to the sensor; the sandwich assay, in which an analyte binds to the sensor followed by the binding of a moiety containing the label; and the competitive or inhibition assay in which an analyte competes with the sensor surface for binding to a labeled moiety.

The direct assay is used for analytes that are easily directly labeled such as PCR amplicons in nucleic acid assays. The sandwich assay is most well-known for giving a low detection limit and high specificity. However, it requires a target analyte to have available sites for the binding of two moieties. Small molecules such as drugs, metabolites, hormones, poisons (e.g. toxins) and vitamins cannot accommodate two binding moieties and thus the competitive assay is preferred. In the case of the sandwich assay the amount of labels detected is directly related to the concentration of the target on the sensor whereas in the case of competitive assay, the amount of label is related to the concentration of free binding sites on the sensor, which decreases with analyte concentration.

The limit of sensitivity of an assay is given by:

$$R_{det} = 2*s.d_b$$

where $R_{det}$ is the limit of sensitivity, and $s.d_b$ is the standard deviation of the signal resulting from the instrumental background and non-specific binding. In order to increase the sensitivity of the assay and thus be able to detect lower concentrations, the signal at these concentrations must be at least $R_{det}$, preferably much higher. The signal detected by a magnetic label is proportional to the density of labels on the sensor and the volume of the magnetic content on the label. Increasing the density of labels bound to the sensor can be achieved by enhancing the efficiency of binding between the components of the assay and by improving the speed and effectiveness of label binding to the surface. However, by increasing the magnetic volume of labels by using large labels (>1$\mu$m diameter) in an assay would increase the contribution in signal per label, but is disadvantageous, since the final stage in the assay requires the binding of the magnetic label on the sensor surface. This process can be very slow and inefficient for larger labels due to steric hindrance and to the reduced surface (for binding to the sensor surface) to volume ratio of larger labels compared to smaller labels. As a consequence even though the signal per label increases with label diameter, the number of labels that contribute to the signal decreases.

BRIEF DESCRIPTION OF THE INVENTION

The object of the present invention is to overcome the above mentioned drawbacks by increasing the sensitivity of an assay and thus enable a detection of lower concentrations.

According to one aspect the present invention relates to a device for amplifying a signal generated from primary nanoparticle labels in an assay, comprising:

-    a separation means for maintaining at least secondary nanoparticle labels separated from the primary nanoparticle labels, wherein the secondary nanoparticle labels are adapted to bind to the primary nanoparticle labels, and
-    a control unit for controlling the releasing of the secondary labels into the assay.

Thereby, a device is provided that is capable of amplifying the generated signal produced by the nanoparticle labels since the density of the labels to be detected, and thus the signal, is increased because one or more secondary labels can bind to a single primary label. It follows that low concentrations of analyte, e.g. <nM, may easily be detected. A further advantage is that the primary labels attaching to the detection surface can be small, thereby reducing the steric hindrance and increasing the surface area per gram of label that can bind to the biosensor surface. The primary labels may be labels that are bound to a target in a solution, or targets attached to a biosensor surface. This binding may take place through intermediate binding groups. According to the present invention, the term nanoparticle label may include particles in the micrometer range, but typically the particles are from several hundred nanometers down to few nanometers, or even a fraction of a nanometer. Also, the geometry of the nanoparticles can be various. The term separation means according to the present invention means physical separation, e.g. where the primary or the secondary labels are in a reservoir, or a chemical separation, i.e. the primary and the secondary labels can not bind together.

In an embodiment, the separation means is selected from a group consisting of:

- a reservoir that is physically isolated from a chamber containing the primary or the secondary nanoparticle labels,
- a second surface adapted to host the secondary nanoparticle labels via external force fields or via chemical binding force, and a force mechanism for applying the external force,
- a second surface adapted to host the secondary nanoparticle labels via external force fields or via chemical binding force, an encapsulating layer of inert labels for generating an inert layer covering the surface of the secondary nanoparticle labels on the second surface, and a force mechanism for applying the external force,
- an encapsulation means and an encapsulate remover for releasing the labels from the encapsulation means, and
- a reservoir comprising a complex containing the binding means for providing the binding member necessary for binding the primary and the secondary labels together, and
- a second surface comprising a complex containing the binding means for providing the binding member necessary for binding the primary and the secondary labels together and an encapsulation means for encapsulating the complex and encapsulation remover for removing the encapsulation means from the complex.

In an embodiment, the primary nanoparticle labels comprise two or more different types of nanoparticle labels in a multi-analyte assay.

In an embodiment, the at least secondary nanoparticle labels comprise additionally tertiary nanoparticle labels, quaternary nanoparticle labels etc. that are separated from each other, wherein the tertiary nanoparticle labels are adapted to bind the secondary nanoparticle labels, the quaternary nanoparticle labels to the are adapted to bind the tertiary nanoparticle labels etc. Since the number of binding sites for the secondary label to the primary label is limited, additional bindings of tertiary labels to the secondary label etc. will allow further amplification of the sensor signal.

In an embodiment, the at least one secondary nanoparticle labels comprises one or more different types of secondary nanoparticle labels. In that way, the secondary nanoparticle labels are capable of binding to various binding types of labels or to various binding sites on the same primary label.

In an embodiment, the nanoparticle labels are magnetic labels, the device further comprising magnetic field producer for generating a magnetic field and thereby inducing magnetic moments in the labels and a biosensor including a surface for detecting the field produced by the labels. Magnetic labels have the advantage that they can be actuated in a magnetic field. In this way they can be actively moved from one location to another. This property can be used for example to enhance the speed at which particles reach the sensor surface, though an attractive force, and for removal of labels that are not bound or are weakly bound to the sensor surface, through a force in the opposite direction. Biosensors that detect magnetic moment have the additional advantage that biological matrices are hardly magnetic and thus do not contribute to background signals.

In an embodiment, the biosensor comprises a GMR, TMR, AMR, or Hall device for detecting the produced field.

In an embodiment, the labels are selected from a group consisting of: metal nanoparticles, semi conducting nanoparticles, polymeric nanoparticles containing a dye, carbon nanoparticles, and magnetic particles containing a dye label.

According to another aspect, the present invention further relates to a method of amplifying a signal generated from a primary nanoparticle labels in an assay, the method comprising:

- maintaining at least secondary nanoparticle labels separated from the primary nanoparticle labels, wherein the secondary nanoparticle labels are adapted to bind to the primary nanoparticle labels, and
- controlling the release of the secondary labels into the assay and allowing the secondary label to bind to the primary labels.

Accordingly, due to the large overall signal contribution per primary label, a low concentration of analyte (<nM) can easily be detected.

In an embodiment, the assay is selected from a group consisting of:

- a sandwich assay,
- a direct assay, and
- a competitive or inhibition assay,
- nucleic acid assays, and
- enzyme activity assays.

Accordingly, generated signal produced by the nanoparticle labels can be amplified independent of the type of assay.

In an embodiment, the diameter of the secondary label is smaller than that of the primary label. It follows that steric hindrance is further reduced. However, the diameter of the secondary label can just as well be similar as that for the primary label, or even larger.

In an embodiment, the primary labels are bound to a surface of a biosensor comprised in the assay.

In an embodiment, the releasing of the secondary labels into the assay is performed subsequently after the primary labels are bound to a surface of a biosensor comprised in the assay.

In an embodiment, the releasing of the secondary labels into the assay is performed subsequently after the primary labels are bound to a surface of a biosensor comprised in the assay and subsequently after unbound or weakly bound primary labels to the biosensor are removed.

In an embodiment, prior to detection the generated signal the primary and the secondary labels that are unbound or weekly bound to a surface of a biosensor comprised in the assay are removed form the assay.

According to still another aspect, the present invention also relates to a use of a combination of a primary and secondary nanoparticle labels for amplifying a signal generated from the primary nanoparticle label in an assay, wherein the at least one secondary nanoparticle label are adapted to be attached to the to the primary labels and thereby act as an amplifying agent.

In an embodiment the primary and at least one secondary nanoparticle labels are magnetic labels and wherein the signal is a magnetic field produced by the labels.

In an embodiment, the use comprises detecting drugs selected from a group consisting of; cannabis, ecstasy, methamphetamine, methadone and amphetamine, cocaine, crack and heroin in a body fluid sample.

In an embodiment, the use comprises detecting proteins, small molecules such as glucose, hormones, toxins, steroids, vitamins and metabolites, peptides, nucleic acids such as DNA and RNA.

The aspects of the present invention may each be combined with any of the other aspects. These and other aspects of the invention will be apparent from and elucidated with reference to the embodiments described hereinafter.

BRIEF DESCRIPTION OF THE DRAWINGS

Embodiments of the invention will be described, by way of example only, with reference to the drawings, in which

Fig. 1 shows a device according to the present invention for amplifying a signal generated from primary nanoparticle labels in an assay by using secondary nanoparticle labels,
Fig. 2 shows an embodiment of the device in Fig. 1,
Fig. 3 illustrates graphically the functional steps performed in the device in Fig. 2,
Fig. 4 shows another embodiment of a device shown in Fig. 1,
Fig. 5 illustrates graphically the functional steps performed in the device in Fig. 4,
Fig. 6 shows another embodiment of a device shown in Fig. 1,
Fig. 7 illustrate graphically the functional steps performed in the device in Fig. 6,
Fig. 8 shows another embodiment of a device shown in Fig. 1,
Fig. 9 illustrate graphically the functional steps performed in the device in Fig. 8,
Fig. 10 shows another embodiment of a device shown in Fig. 1,
Fig. 11 illustrate graphically the functional steps performed in the device in Fig. 10, and
Fig. 12 shows a flow diagram of a method of amplifying a signal generated from primary nanoparticle labels in an assay.

DESCRIPTION OF EMBODIMENTS

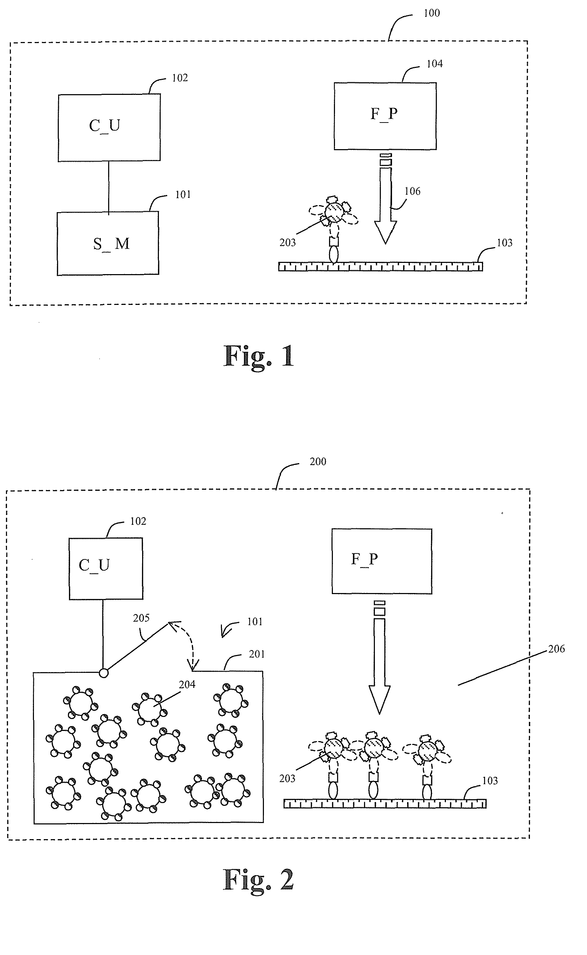

Fig. 1 shows a device 100 according to the present invention for amplifying a signal generated from primary nanoparticle labels in an assay by using secondary nanoparticle labels. In the following embodiment, it is assumed that the nanoparticle labels comprise magnetic labels, but other types of labels are possible, such as metal nanoparticles, semi conducting nanoparticles, polymeric nanoparticles containing a dye, carbon nanoparticles, and magnetic particles containing a dye label.

The amplification is obtained by attaching one or more of the secondary magnetic labels to the primary magnetic labels. As shown, the device comprises separation means (S_U) 101, a control unit (C_U) 102, typically a magnetic sensor, a magnetic field producer (F_P) 104 and a biosensor 103 including a surface for detecting the field produced by the labels 203. The sensor can be any suitable sensor to detect the presence of magnetic particles on or near to a sensor surface, based on any property of the particles, e.g. it can detect via magnetic methods (e.g. magnetoresistive, Hall, coils), optical methods (imaging, fluorescence, chemiluminescence, absorption, scattering, surface plasmon resonance, Raman, etc.), sonic detection (surface acoustic wave, bulk acoustic wave, cantilever, quartz crystal etc), electrical detection (e.g. conduction, impedance, amperometric, redox cycling), etc. The separation means (S_U) 101 is adapted for separating the secondary labels from the primary labels and in that way seal off the secondary labels from the biosensor 103. The separation may comprise a physical separation, e.g. in the form of a reservoir, or a chemical separation where the labels can not be chemically bound together. The control unit (C_U) 102 is adapted to control the releasing of the secondary labels from the reservoir separation means (S_M) 101. The field producer (F_P) 104 can either be an internal or an external unit and can comprise wires where the magnetic field produced is produced by the current in the wires, electromagnetic coils, and permanent magnets. The field producer is adapted to generate a magnetic field 106 in order to induce magnetic moments in the labels 203, 204. The biosensor 103 then detects the field produced 107 by the labels, e.g. by using Giant Magnetor Resistive element (GMR), Tunneling Magnetic Resistive element (TMR), Anisotropic Magneto Resistive element (AMR), Hall element, and the like.

Fig. 2 shows an embodiment of a device 200 according to the present invention for amplifying a signal generated from primary labels in an assay by using secondary magnetic labels. In this embodiment the separation means (S_M) 101 includes a reservoir or chamber 201 for holding the secondary labels 204 physically separated from the primary labels 203. The reservoir 201 further contains a gate 205, or mechanical, electrical or magnetically active valve, operated by the control unit (C_U) 102 for allowing a flow of the secondary particles 204 into the main chamber 206. In an embodiment, the control unit (C_U) 102 is computer controlled wherein the controlling may e.g. comprise introducing only a portion of secondary labels into the main chamber 206 where the biosensor 103 is placed. Accordingly, the computer (not shown) could be adapted to utilize physical parameters such as the pressure in the reservoir 201 to approximate the number of secondary labels 202, and to optimize the time of opening to control the outflow of the secondary particles.

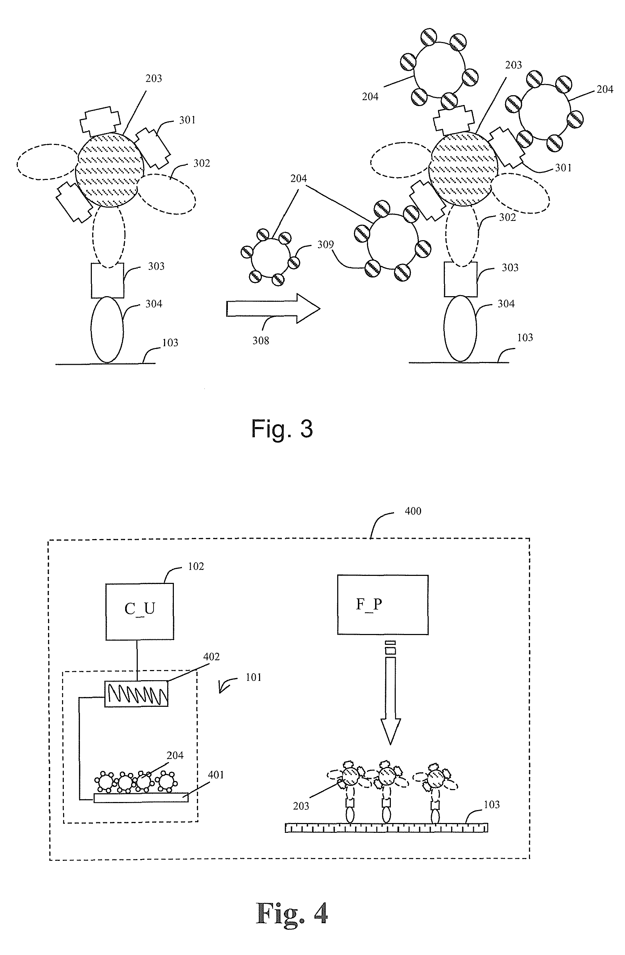

Fig. 3 illustrates graphically the functional steps performed in the device in Fig. 2 from where the primary labels 204 are attached to the surface of the biosensor 103 in the absence of the secondary labels 204 (the left side of Fig. 3), until the secondary labels 204 have been released from the reservoir 201 and are attached to the primary labels (the right side of Fig. 3). In this embodiment, the primary labels comprises two binding moieties 301, 302, one moiety 302 for binding the primary label to the target 303, wherein the target 303 then binds to the surface via binding moiety 304, and one binding moiety 301 for binding with the secondary label 204. The number of binding moieties of the primary label could just as well comprise one binding moiety or more than two binding moieties. The binding moieties can include avidin, biotin, hapten, antibody, protein, peptide, lectin, carbohydrate, aptamer, and nucleic acid.

The diameter of the primary magnetic labels may be few nanometers up to several hundred nanometers, or even up to the micron range. In an embodiment, the nanoparticles labels are made of polymer enclosing small grains of iron oxide and form a matrix of iron oxide, and therefore have super-paramagnetic properties. The material properties of the magnetic labels could however be such that the labels have para- or ferromagnetic properties.

The binding moieties on the surface of the primary 204 and the secondary 203 labels are in one embodiment achieved by functionalizing the surface of the labels 203, 204 by e.g. coating them with or carrying out surface reactions to create reactive functional groups such as carboxylic acid, amine, tosyl, aldehyde, maleimide, thiol or epoxy. The result of such coating provides the reactive groups for the immobilization of binding moieties including biological molecules such as avidin, biotin, antibodies, peptides, aptamers, and oligonucleotides. Such binding moieties can also be modified so that they react more easily with the reactive groups on the surface. For example carboxylic acid groups react with amine groups which can occur naturally on proteins and peptides but these amine groups can also be added to oligonucleotides and aptamers to enable their immobilization on carboxylated surfaces.

It should be noted that the embodiment shown here is so-called sandwich assay where the target 303 is sandwiched between moiety 304 on the biosensor surface 103 and moiety 302 that is coupled to the primary label 203, i.e. the target analyte must have two available sites for binding. The scenario as illustrated in Fig. 3, as well as other embodiments described later, could just as well apply for other types of assays, e.g. direct assay, in which a labeled analyte binds to the to the sensor followed by the binding of a moiety containing the primary label 204, or competitive or inhibition assay which is preferred for e.g. molecules such as drugs, metabolites, hormones, poisons (e.g. toxins) and vitamins which that cannot accommodate two binding moieties.

The secondary label 204 comprises in this embodiment one type of binding moiety 309 that is suitable to bind with the binding moiety 301 on the primary label. The number of binding moieties on the secondary label 204 could of course comprise more than one type of binding moieties, wherein each respective binding moiety could be adapted to a specific binding moiety on the primary label (and also tertiary moiety).

The arrow 308 illustrates the opening of the reservoir, i.e. releasing of the secondary labels 204 into the main chamber 206. The right side of Fig. 3 shows where the secondary labels 204 are attached to the primary label 203 via the binding moiety 301. Having these second labels 204 attached to the primary label will result in that the signal generated by the primary label 203 will be amplified and therefore the sensitivity of the assay will be increased due to the enlarged density of labels associated to each respective target 303.

Fig. 4 shows another embodiment of a device 400 according to the present invention for amplifying a signal generated from primary labels 203 in an assay by using secondary magnetic labels 204, where the primary 203 and the secondary 204 labels are placed within the same chamber. In this embodiment the separation means (S_M) 101 comprises a second surface 401 to which the secondary labels 204 are attached to. The attachment can be accomplished by applying a magnetic field at a specific location, e.g. on surface 401, and utilize the magnetic properties of the secondary labels 204 to maintain them at the surface 401. By removing the magnetic field the secondary labels 204 will be released from the surface, whereby the control unit (C_U) 102 comprises mechanism 402, preferably a computer controlled, for supplying the magnetic field 302 and for releasing the magnetic field locally. Another way of attracting the secondary labels to the surface could be by using electrostatic attraction, wherein the step of releasing the labels from the surface 401 could comprises an application of a current or voltage to the surface 401 at a level and sign that removes the electrostatic attraction force and/or provides an electrostatic repulsive force.

Furthermore, to speed up binding of the secondary labels 204, they can be attracted to the desired location containing the primary labels 203, i.e. the sensor surface 103 with a magnetic field. Removal of unbound or weakly bound secondary labels 204 can be achieved with a magnetic force away from the sensor surface 203. Furthermore, the type of label to be attracted to a certain location can be discriminated by the frequency of the actuation field. Labels of a particular type can be magnetized at higher filed frequencies then labels of another type. By using high frequency field it is possible to attract one type of labels, while not affecting labels of another type. The detection of the particles would typically be done using a field frequency where both labels are magnetized.

Instead of using magnetic field and utilize the magnetic properties, the separation means (S_M) 101 could just as well comprise a surface having such surface properties that the secondary labels 204 will be attached to, wherein the step of releasing the labels from the surface 401 comprises supplying heat energy to the surface 401 (such that the energy followed by the heat exceeds the binding energy between the label and the surface). Accordingly, a computer controlled heater may just as well be implemented to control the releasing of the secondary labels 204. An additional method to attract the secondary labels 204 to the surface 401 can be due to electrostatic attraction, wherein the step of releasing the labels from the surface 401 comprises application of a current or voltage to surface 401 at a level and sign that removes the electrostatic attraction force and/or provides an electrostatic repulsive force.

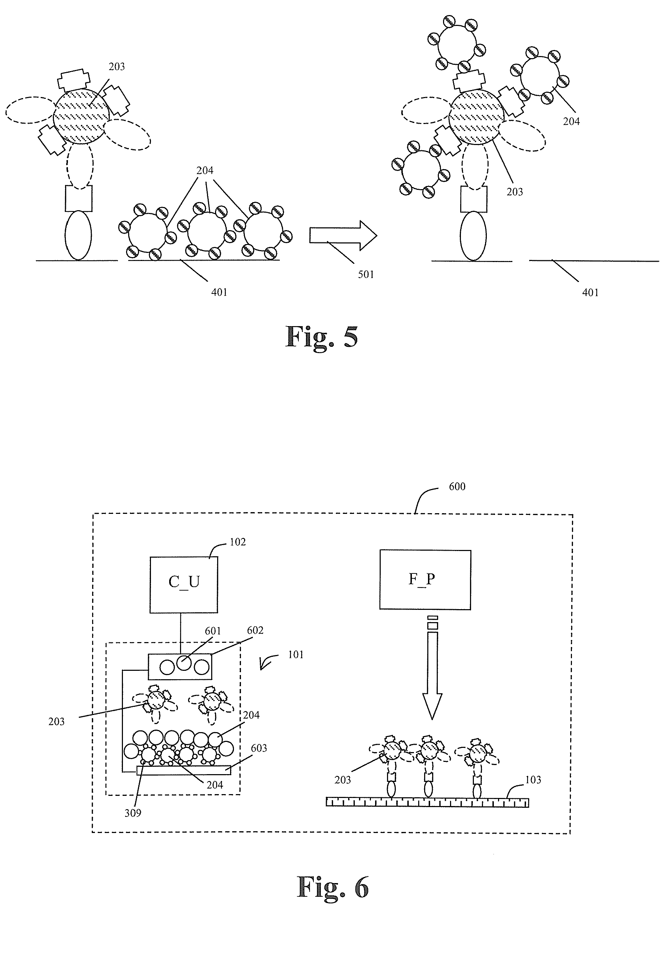

Fig. 5 illustrate graphically the functional steps from where the primary labels 203 in Fig. 4 are attached to the surface 103 in the absence of the secondary labels 204 (the left side of Fig. 5), until the secondary labels 204 have been released from the surface 401 and are attached to the primary labels (the right side of Fig. 5). The arrow 501 may stand for the previously application of a magnetic field or heat energy that causes the releasing of the secondary labels 204 from the surface 401.

Fig. 6 shows a still another embodiment of a device 600 according to the present invention for amplifying a signal generated from primary labels in an assay by using secondary magnetic labels 204, wherein the separation means (S_M) 101 comprises a second surface 603 to which the secondary magnetic labels 204 are bound to via e.g. chemical binding or attracted to via external force to surface 603 such a magnetic or electric force. In this embodiment, secondary inert labels or any other kinds of inert labels or particles having no binding members are used to provide an inert layer on the secondary labels 204. The inert secondary labels 204 may be attracted to the surface 603 where the primary labels 204 are by e.g. applying a magnetic field, wherein the same magnetic field may be adapted to keep both the "active" (comprising binding members) secondary labels and the inert secondary labels 204. The result thereof is that the primary labels 203, which have not yet bound to the biosensor surface 103 cannot bind to the secondary labels, protected by the inert layer, and the secondary labels can be placed within the same chamber. The releasing of the secondary labels 204, both those having no binding members and those having binding member could be realized by releasing or changing the field. The role of the control unit (C_U) 102 in this embodiment could comprise controlling the electric or the magnetic field.

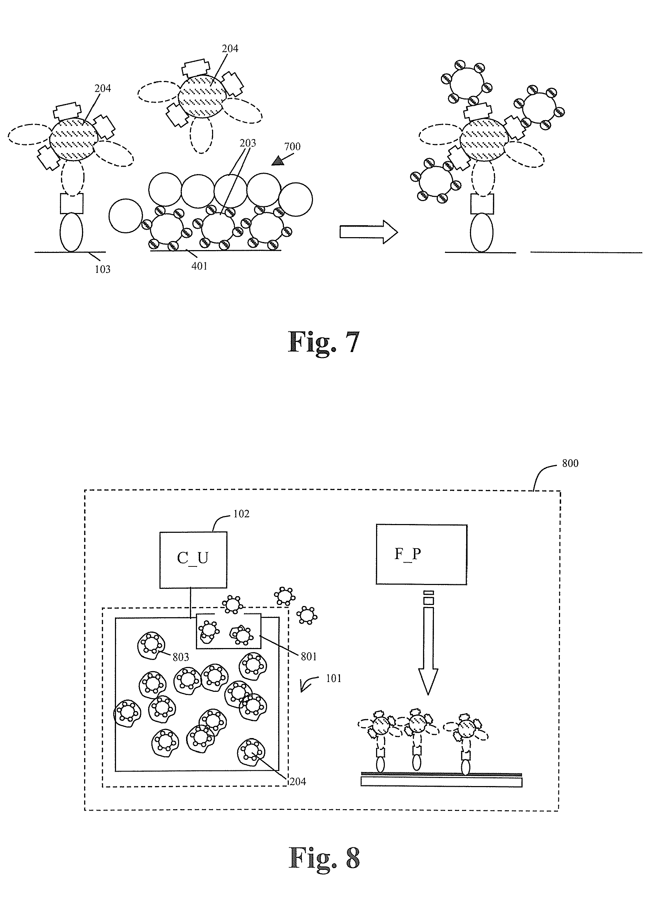

Fig. 7 illustrate graphically the functional steps performed in the device in Fig. 6 where the left side shows the inert layer 700 formed by the inert secondary labels 204 and primary labels 204 surrounding the secondary labels 203. The right side of Fig. 7 shows where the inert labels 204 have been released from the surface by e.g. releasing the magnetic field.

Fig. 8 shows yet another embodiment of a device 800 according to the present invention for amplifying a signal generated from primary labels in an assay by using secondary magnetic labels. In this embodiment, the separation means comprises an encapsulation means 803, and encapsulate remover 801 having a couple to a material that can be magnetically, mechanically, chemically or electrically activated to dissolve or decompose and thus release the label from the encapsulation means 803. The encapsulation process can be done in-situ in the device 800, or externally. If as an example, the secondary labels 204 are encapsulated in a polymer, it will dissolve or melt upon the application of heat (e.g. gels such as low melting point agarose, waxes, and hydrogen-bonded polymers). Accordingly, the encapsulate remover 801 could comprise a heater for supplying the heat. In an embodiment, the secondary labels can be encapsulated by placing them in a polymer or lipid capsule than can be forced open upon exposure of an ultrasound pulse. The encapsulate means can cover individual nanoparticles or groups of nanoparticles. The control unit (C_U) 102 is accordingly adapted to control the encapsulation remover 801 and the couple to the material.

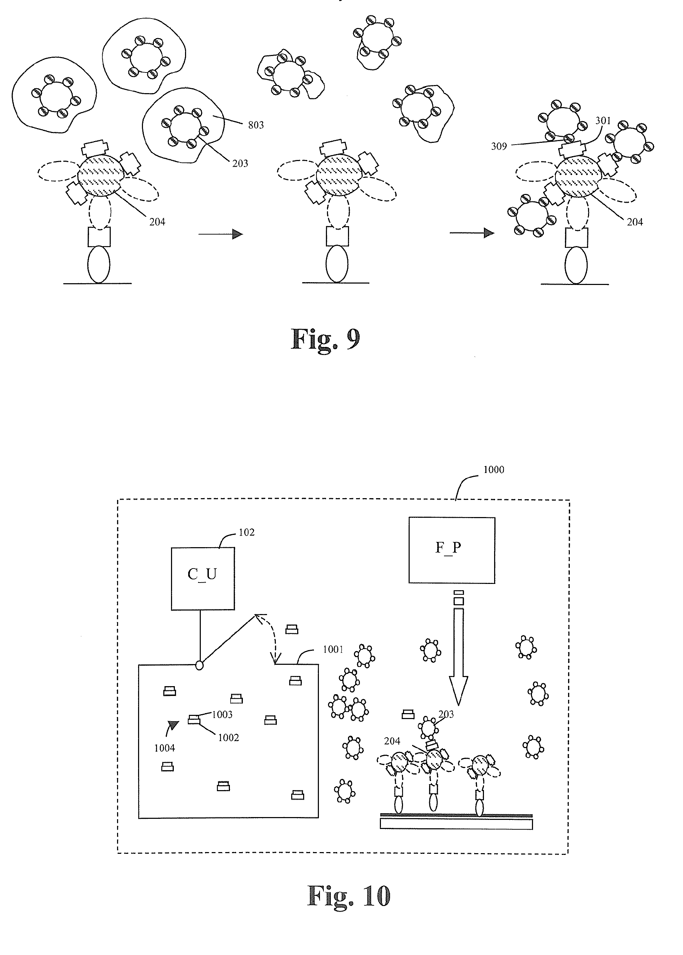

Fig. 9 illustrate graphically the functional steps performed in the device in Fig. 8 where initially the secondary labels 203 are encapsulated in the encapsulation means 803, until the encapsulation means 803 has been removed and the binding moiety 309 is exposed and can bind to moiety 310 on the primary label 204.

Fig. 10 shows still another embodiment of a device 1000 according to the present invention for encapsulating the secondary labels for amplifying a signal generated from primary labels in an assay by using secondary magnetic labels. In this embodiment, the separation means comprises a complex 1004 that could initially be kept separated from the labels in a reservoir 1001, where the complex contains at least a first moiety 1002 for binding the primary labels and a second moiety 1003 for binding to the secondary label 203. In this embodiment, the primary labels comprise no binding moieties that enable them to bind directly to the secondary label. Accordingly, the complex 1004 provides a binding between the primary and the secondary labels 203, 204. The result thereof is that the primary and the secondary labels can be placed within the same chamber.

The complex can be released from a physically isolated reservoir 1001 as shown here or activated and released from an encapsulation layer similar to that as described previously. The control unit (C_U) 102 may be adapted to control the reservoir 1001, e.g. by controlling the flow of the complexes 1002 from the reservoir into the main chamber.

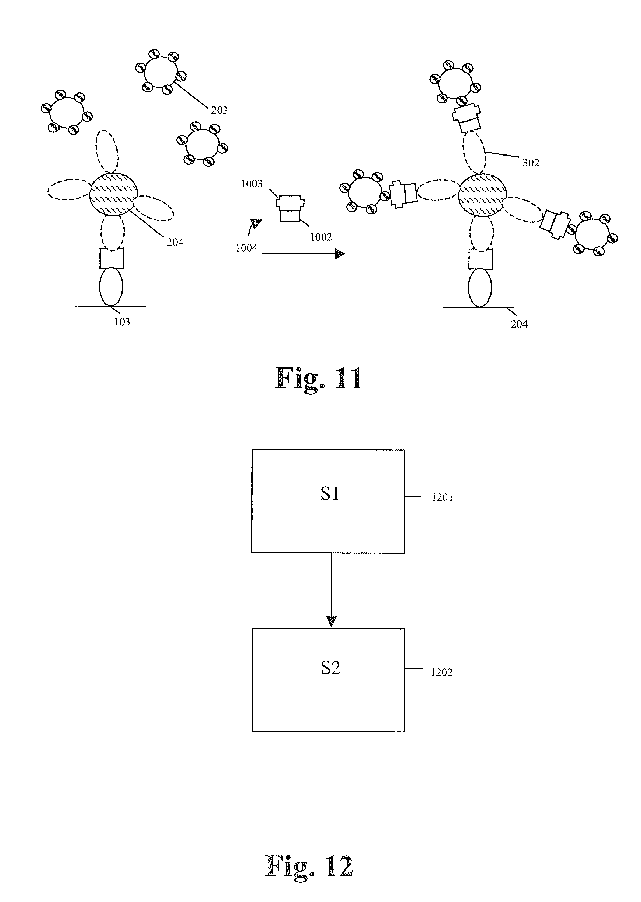

Fig. 11 illustrate graphically the functional steps performed in the device in Fig. 10, showing the primary label 204 with only one type of binding moiety 302, whereby after the addition of the complex 1004 the secondary label 203 can bind to the primary label 204 via the binding moieties 1002, 1003.

Fig. 12 shows a flow diagram of a method of amplifying a signal generated from primary nanoparticle labels in an assay by using secondary nanoparticle labels. The assay may comprise a sandwich assay, a direct assay, a competitive or inhibition assay and the like. Initially, the secondary nanoparticle labels separated from the primary nanoparticle labels (S1) 1201. This may be done to allow the primary labels to initially bind to a biosensor surface. The primary labels that have not bound or are bound weakly may be thereafter removed through washing with a force (e.g. magnetic or hydrodynamic) in order to prevent the amplification of non-specifically bound primary labels (i.e. labels that are not attached to a target). Subsequently, the secondary labels are released into the assay comprising the primary labels (S2) 1202, wherein the releasing of the secondary labels is done in a controlled away. The secondary labels bind to the primary labels and secondary labels that are not bound or are weakly bound to the primary labels may be removed.

Certain specific details of the disclosed embodiment are set forth for purposes of explanation rather than limitation, so as to provide a clear and thorough understanding of the present invention. However, it should be understood by those skilled in this art, that the present invention might be practiced in other embodiments that do not conform exactly to the details set forth herein, without departing significantly from the spirit and scope of this disclosure. Further, in this context, and for the purposes of brevity and clarity, detailed descriptions of well-known apparatuses, circuits and methodologies have been omitted so as to avoid unnecessary detail and possible confusion.

Reference signs are included in the claims, however the inclusion of the reference signs is only for clarity reasons and should not be construed as limiting the scope of the claims.

## Claims

1. A device (100) for amplifying a signal generated from primary nanoparticle labels (204) in an assay, comprising:

    - a separation means (101) for maintaining at least secondary nanoparticle labels (203) separated from the primary nanoparticle labels, wherein the secondary nanoparticle labels (203) are adapted to bind to the primary nanoparticle labels (204), and
    - a control unit (102) for controlling the releasing of the secondary labels into the assay.

2. A device according to claim 1, wherein the separation means (101) is selected from a group consisting of :

    - a reservoir (201) that is physically isolated from a chamber containing the primary (204) or the secondary (203) nanoparticle labels,
    - a second surface (401) adapted to host the secondary nanoparticle labels via external force fields or via chemical binding force, and a force mechanism for applying the external force,
    - a second surface (603) adapted to host the secondary nanoparticle labels via external force fields or via chemical binding force, an encapsulating layer of inert labels for generating an inert layer (701) covering the surface of the secondary nanoparticle labels (203) on the second surface (603), and a force mechanism for applying the external force,
    - an encapsulation means (803) and an encapsulate remover (801) for releasing the labels from the encapsulation means, and
    - a reservoir (1001) comprising a complex (1004) containing the binding means for providing the binding member necessary for binding the primary (204) and the secondary labels (203) together, and
    - a second surface comprising a complex (1004) containing the binding means for providing the binding member necessary for binding the primary and the secondary labels together and an encapsulation means for encapsulating the complex and encapsulation remover for removing the encapsulation means from the complex.

3. A device according to claim 1, wherein the primary (204) nanoparticle labels comprise two or more different types of nanoparticle labels in a multi-analyte assay.

4. A device according to claim 1, wherein the at least secondary nanoparticle labels (203) comprise additionally tertiary nanoparticle labels, quaternary nanoparticle labels etc. that are separated from each other, wherein the tertiary nanoparticle labels are adapted to bind the secondary nanoparticle labels, the quaternary nanoparticle labels to the are adapted to bind the tertiary nanoparticle labels etc.

5. A device according to claim 1, wherein the at least one secondary nanoparticle labels (203) comprises one or more different types of secondary nanoparticle labels.

6. A device according to claim 1, wherein the nanoparticle labels (203, 204) are magnetic labels, the device further comprising magnetic field producer (F_P) 104 for generating a magnetic field 106 and thereby inducing magnetic moments in the labels and a biosensor (103) including a surface for detecting the field produced by the labels (203, 204).

7. A device according to claim 6, where the biosensor (103) comprises a GMR, TMR, AMR, or Hall device for detecting the produced field.

8. A device according to claim 1, wherein the labels are selected from a group consisting of: metal nanoparticles, semi conducting nanoparticles, polymeric nanoparticles containing a dye, carbon nanoparticles, and magnetic particles containing a dye label.

9. A method of amplifying a signal generated from primary nanoparticle labels (204) in an assay, the method comprising:

   - maintaining (1201) at least secondary nanoparticle labels (203) separated from the primary nanoparticle labels, wherein the secondary nanoparticle labels (203) are adapted to bind to the primary nanoparticle labels (204), and
   - controlling (1202) the release of the secondary labels (203) into the assay.

10. A method according to claim 9, wherein the assay is selected from a group consisting of:

   - a sandwich assay,
   - a direct assay, and
   - a competitive or inhibition assay,
   - nucleic acid assays, and
   - enzyme activity assays.

11. A method according to claim 9, wherein the diameter of the secondary labels (203) is smaller than that of the primary labels (204).

12. A method according to claim 9, wherein the primary labels (204) are bound to a surface of a biosensor (103) comprised in the assay.

13. A method according to claim 9, wherein the releasing of the secondary labels (203) into the assay is performed subsequently after the primary labels (204) are bound to a surface of a biosensor (103) comprised in the assay.

14. A method according to claim 9, wherein the releasing of the secondary labels (203) into the assay is performed subsequently after the primary labels (204) are bound to a surface of a biosensor (103) comprised in the assay and subsequently after unbound or weakly bound primary labels to the biosensor (103) are removed.

15. A method according to claim 9, wherein prior to detection the generated signal the primary (204) and the secondary labels (203) that are unbound or weekly bound to a surface of a biosensor (103) comprised in the assay are removed form the assay.

16. A use of a combination of primary nanoparticle label and secondary nanoparticle labels for amplifying a signal generated from the primary nanoparticle label in an assay, wherein the at least one secondary nanoparticle label are adapted to be attached to the primary labels and thereby act as an amplifying agent.

17. A use according to claim 16 for detecting drugs selected from a group consisting of; cannabis, ecstasy, methamphetamine, methadone and amphetamine, cocaine, crack and heroin in a body fluid sample.

18. A use according to claim 16 for detecting proteins, small molecules such as glucose, hormones, toxins, steroids, vitamins and metabolites, peptides, nucleic acids such as DNA and RNA.

**Fig. 1**

**Fig. 2**

Fig. 3

Fig. 4

Fig. 5

Fig. 6

**Fig. 7**

**Fig. 8**

803

203

204

301

309

204

**Fig. 9**

1000

102

C_U

F_P

1001

1003

1002

1004

203

204

**Fig. 10**

**Fig. 11**

**Fig. 12**

**European Patent Office**

# EUROPEAN SEARCH REPORT

Application Number

EP 06 11 5479

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BRUSH M: "Automated liquid handlers advance"<br>THE SCIENTIST,<br>vol. 16, no. 3,<br>4 February 2002 (2002-02-04), page 38,<br>XP002407186<br>* the whole document * | 1-5,8 | INV.<br>G01N33/543<br>G01N33/58 |
| X | WO 02/46472 A2 (NANOSPHERE INC; MIRKIN C A; LETSINGER R L; MUCI)<br>13 June 2002 (2002-06-13)<br>* the whole document *<br>in particular p.39, l.17-22; p.41, l.27 to p.43, l.4; p.70, l.15-25; p.73, l.3-8; p.97, l.11-17; ex.10+16; fig.13+25. | 1-5,<br>8-10,<br>12-18 | |
| X | WO 00/28088 A (BIOCRYSTAL LTD)<br>18 May 2000 (2000-05-18)<br><br>* the whole document *<br>in particular p.6, l.28 to p.7, l.12;<br>claims 14,15,21,22; fig.3-8. | 1-5,<br>8-10,<br>12-18 | |
| X | WO 02/31191 A2 (YISSUM RES DEV CO; PATOLSKY F; WILLNER I)<br>18 April 2002 (2002-04-18)<br>* the whole document *<br>in particular claims 1-6,8,9,13,17;<br>fig.1+2; ex.1+2. | 1,2,<br>8-10,<br>12-16,18 | TECHNICAL FIELDS SEARCHED (IPC)<br><br>G01N<br>G01R<br>B01L |

-/--

The present search report has been drawn up for all claims

| Place of search | Date of completion of the search | Examiner |
|---|---|---|
| The Hague | 11 April 2007 | Weber, Peter |

EPO FORM 1503 03.82 (P04C01)

**European Patent Office**

**EUROPEAN SEARCH REPORT**

Application Number

EP 06 11 5479

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JEON H ET AL: "Vesicle-reconstituted low density lipoprotein receptor. Visualization by cryoelectron microscopy." J BIOL CHEM, vol. 275, no. 39, 29 September 2000 (2000-09-29), pages 30458-30464, XP002407188 ISSN: 0021-9258 * the whole document * in particular abstract; p.30460, col.1, par.3. | 1,2, 8-16,18 | |
| X | GIFFIN B F ET AL: "Enhancement of antigenic site detection with gold labeled secondary and tertiary antibodies using the immunogold-silver staining method." BIOTECHNIC & HISTOCHEMISTRY, vol. 68, no. 6, November 1993 (1993-11), pages 309-315, XP008071261 ISSN: 1052-0295 * the whole document * in particular fig.3. | 1,2, 8-16,18 | |
| X | LI JIONG ET AL: "Amplifying the electrical hybridization signals of DNA array by multilayer assembly of Au nanoparticle probes." THE ANALYST, vol. 128, no. 7, July 2003 (2003-07), pages 917-923, XP002407190 ISSN: 0003-2654 * the whole document * in particular fig.1. | 1,2, 8-16,18 | TECHNICAL FIELDS SEARCHED (IPC) |

-/--

The present search report has been drawn up for all claims

| Place of search | Date of completion of the search | Examiner |
|---|---|---|
| The Hague | 11 April 2007 | Weber, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

**EUROPEAN SEARCH REPORT**

Application Number

EP 06 11 5479

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHU, XIA ET AL: "Quartz crystal microbalance immunoassay with dendritic amplification using colloidal gold immunocomplex" SENSORS AND ACTUATORS, B, vol. 114, no. 2, 2 August 2005 (2005-08-02), pages 696-704, XP002407191 * the whole document * in particular scheme 2. | 1,2, 8-16,18 | |
| X | HAZARIKA, P ET AL: "Sensitive detection of proteins using difunctional DNA-gold nanoparticles" SMALL, vol. 1, no. 8-9, 2005, pages 844-848, XP002407187 * the whole document * in particular fig.1c. | 1-5, 8-16,18 | |
| X | TAMANAHA C R ET AL: "Hybrid macro-micro fluidics system for a chip-based biosensor" JOURNAL OF MICROMECHANICS & MICROENGINEERING, vol. 12, no. 2, 1 March 2002 (2002-03-01), pages N7-N17, XP020068749 * the whole document * | 1-8 | **TECHNICAL FIELDS SEARCHED (IPC)** |
| X | WO 01/41736 A (MASSACHUSETTS INST TECHNOLOGY) 14 June 2001 (2001-06-14) * the whole document * | 1,2 | |
| X | WO 01/12157 A (MICROCHIPS INC) 22 February 2001 (2001-02-22) * the whole document * | 1,2 | |

-/--

The present search report has been drawn up for all claims

| Place of search | Date of completion of the search | Examiner |
|---|---|---|
| The Hague | 11 April 2007 | Weber, Peter |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    ............................................................................................
& : member of the same patent family, corresponding
    document

EPO FORM 1503 03.82 (P04C01)

**European Patent Office**

**EUROPEAN SEARCH REPORT**

Application Number

EP 06 11 5479

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 98/00107 A (MASSACHUSETTS INST TECHNOLOGY [US]) 8 January 1998 (1998-01-08) * the whole document * | 1,2 | |
| X | WO 2005/016558 A (MICROCHIPS INC; COPPETA J R; MALONEY J M) 24 February 2005 (2005-02-24) * the whole document * | 1,2 | |
| X | US 2005/170418 A1 (MORELAND JOHN [US] ET AL) 4 August 2005 (2005-08-04) * the whole document * | 1,2 | |
| X | KWAKYE ET AL: "Electrochemical microfluidic biosensor for nucleic acid detection with integrated minipotentiostat" BIOSENSORS & BIOELECTRONICS, vol. 21, no. 12, 18 January 2006 (2006-01-18), pages 2217-2223, XP005412598 * the whole document * | 1,2 | |
| X | DE 20 28 822 A1 (KELLER H) 16 December 1971 (1971-12-16) * the whole document * | 1,2 | TECHNICAL FIELDS SEARCHED (IPC) |
| A | GRAHAM D L ET AL: "Magnetoresistive-based biosensors and biochips" TRENDS IN BIOTECHNOLOGY, vol. 22, no. 9, September 2004 (2004-09), pages 455-462, XP004552610 * the whole document * | 1,6,7 | |

The present search report has been drawn up for all claims

| Place of search | Date of completion of the search | Examiner |
|---|---|---|
| The Hague | 11 April 2007 | Weber, Peter |

EPO FORM 1503 03.82 (P04C01)

European Patent
Office

Application Number

EP 06 11 5479

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

**LACK OF UNITY OF INVENTION
SHEET B**

The Search Division considers that the present European patent application does not comply with the requirements of unity of invention and relates to several inventions or groups of inventions, namely:

1. claims: 1,3-5,8-18 (completely) 2 (partially)

   Devices for amplifying a signal generated from primary nanoparticle labels with secondary nanoparticle labels by releasing them in an assay comprising a physically isolated reservoir as a separation means and a control unit. Corresponding methods and uses.
   ---

2. claim: 2 (partially)

   Devices for amplifying a signal generated from primary nanoparticle labels with secondary nanoparticle labels in an assay by releasing them comprising a surface to host compounds relevant for the amplifying interaction as a separation means and a control unit.
   ---

3. claim: 2 (partially)

   Devices for amplifying a signal generated from primary nanoparticle labels with secondary nanoparticle labels by releasing them in an assay comprising encapsulating means as a separation means and a control unit.
   ---

4. claim: 2 (partially)

   Devices for amplifying a signal generated from primary nanoparticle labels with secondary nanoparticle labels by releasing them in an assay comprising a separation means involving a complex containing binding means for the amplifying interaction of the two labels and a control unit.
   ---

5. claims: 6,7 (completely)

   Devices for amplifying a signal generated from primary nanoparticle labels with secondary nanoparticle labels by releasing them in an assay comprising a separation means, a control unit, a magnetic field producer, and a biosensor for detecting fields produced by magnetic labels.
   ---

## ANNEX TO THE EUROPEAN SEARCH REPORT
## ON EUROPEAN PATENT APPLICATION NO.

EP 06 11 5479

This annex lists the patent family members relating to the patent documents cited in the above-mentioned European search report.
The members are as contained in the European Patent Office EDP file on
The European Patent Office is in no way liable for these particulars which are merely given for the purpose of information.

11-04-2007

| Patent document cited in search report | | Publication date | Patent family member(s) | | Publication date |
|---|---|---|---|---|---|
| WO 0246472 | A2 | 13-06-2002 | AU | 3059302 A | 18-06-2002 |
| | | | EP | 1356109 A2 | 29-10-2003 |
| WO 0028088 | A | 18-05-2000 | AU | 1717500 A | 29-05-2000 |
| | | | EP | 1157133 A1 | 28-11-2001 |
| | | | US | 6261779 B1 | 17-07-2001 |
| | | | US | 2002150905 A1 | 17-10-2002 |
| WO 0231191 | A2 | 18-04-2002 | AU | 9415001 A | 22-04-2002 |
| | | | CA | 2424435 A1 | 18-04-2002 |
| | | | EP | 1368490 A2 | 10-12-2003 |
| | | | IL | 138988 A | 25-09-2005 |
| | | | JP | 2005507488 T | 17-03-2005 |
| | | | US | 2004048272 A1 | 11-03-2004 |
| WO 0141736 | A | 14-06-2001 | AT | 323470 T | 15-05-2006 |
| | | | AU | 782639 B2 | 18-08-2005 |
| | | | AU | 4513101 A | 18-06-2001 |
| | | | AU | 2005234663 A1 | 15-12-2005 |
| | | | CA | 2393603 A1 | 14-06-2001 |
| | | | EP | 1235560 A2 | 04-09-2002 |
| | | | JP | 2003516343 T | 13-05-2003 |
| WO 0112157 | A | 22-02-2001 | AT | 290364 T | 15-03-2005 |
| | | | AU | 6639400 A | 13-03-2001 |
| | | | CA | 2381951 A1 | 22-02-2001 |
| | | | DE | 60018582 D1 | 14-04-2005 |
| | | | DE | 60018582 T2 | 19-01-2006 |
| | | | EP | 1210064 A1 | 05-06-2002 |
| | | | JP | 2003506477 T | 18-02-2003 |
| WO 9800107 | A | 08-01-1998 | AT | 216219 T | 15-05-2002 |
| | | | CA | 2258898 A1 | 08-01-1998 |
| | | | DE | 69712063 D1 | 23-05-2002 |
| | | | DE | 69712063 T2 | 24-10-2002 |
| | | | DK | 914092 T3 | 12-08-2002 |
| | | | EP | 0914092 A2 | 12-05-1999 |
| | | | ES | 2176768 T3 | 01-12-2002 |
| | | | JP | 2000513725 T | 17-10-2000 |
| | | | JP | 2006096760 A | 13-04-2006 |
| | | | PT | 914092 T | 30-09-2002 |
| | | | US | 6123861 A | 26-09-2000 |
| | | | US | 5797898 A | 25-08-1998 |
| WO 2005016558 | A | 24-02-2005 | NONE | | |

## ANNEX TO THE EUROPEAN SEARCH REPORT
## ON EUROPEAN PATENT APPLICATION NO.

EP 06 11 5479

This annex lists the patent family members relating to the patent documents cited in the above-mentioned European search report.
The members are as contained in the European Patent Office EDP file on
The European Patent Office is in no way liable for these particulars which are merely given for the purpose of information.

11-04-2007

| Patent document cited in search report | Publication date | Patent family member(s) | Publication date |
|---|---|---|---|
| US 2005170418 A1 | 04-08-2005 | NONE | |
| DE 2028822 A1 | 16-12-1971 | NONE | |

EPO FORM P0459

For more details about this annex : see Official Journal of the European Patent Office, No. 12/82